(19) Europäisches Patentamt
European Patent Office
Office européen des brevets

(11) Veröffentlichungsnummer: **0 509 341 A1**

(12) **EUROPÄISCHE PATENTANMELDUNG**

(21) Anmeldenummer: **92105746.9**

(22) Anmeldetag: **03.04.92**

(51) Int. Cl.[5]: **C07D 277/56**, C07D 417/06, A01N 43/78

(30) Priorität: **16.04.91 DE 4112329**

(43) Veröffentlichungstag der Anmeldung: **21.10.92 Patentblatt 92/43**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Kluth, Joachim, Dr.**
**Tannenweg 9**
**W-4018 Langenfeld(DE)**
Erfinder: **Tietjen, Klaus Günther, Dr.**
**Am Alten Broich 98**
**W-4018 Langenfeld(DE)**
Erfinder: **Jansen, Johannes R., Dr.**
**Knipprather Strasse 47**
**W-4019 Monheim(DE)**
Erfinder: **Kuhnt, Dietmar, Dr.**
**Körnerstrasse 5**
**W-5090 Leverkusen 1(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Cyanothiazolderivate.**

(57) Die Erfindung betrifft neue Cyanothiazolderivate der allgemeinen Formel (I)

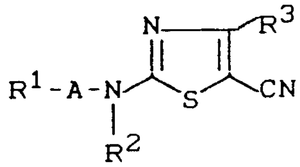

(I)

in welcher
- A für Alkandiyl, Oxaalkandiyl, Alkendiyl oder Alkindiyl steht,
- $R^1$ für jeweils gegebenenfalls substituiertes Cycloalkyl, Aryl oder Heterocyclyl steht,
- $R^2$ für Wasserstoff oder Alkyl steht und
- $R^3$ für Wasserstoff, Halogen oder gegebenenfalls substituiertes Alkyl steht,

ein Verfahren zu ihrer Herstellung und ihre Verwendung als Herbizide.

EP 0 509 341 A1

Die Erfindung betrifft neue Cyanothiazolderivate, ein Verfahren zu ihrer Herstellung und ihre Verwendung als Herbizide.

Es ist bereits bekannt, daß bestimmte 5-Cyano-2,4-diamino-thiazol-Derivate, wie z.B. (S)-4-Amino-5-cyano-2-(1-phenyl-ethylamino)-thiazol herbizide Eigenschaften besitzen (vgl. EP-A 328954). Die Wirksamkeit dieser bekannten Verbindungen ist jedoch nicht in allen Belangen zufriedenstellend.

Es wurden nun neue Cyanothiazolderivate der allgemeinen Formel (I)

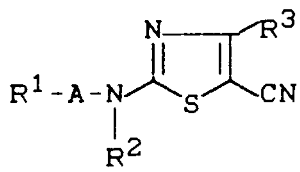

(I)

in welcher

A für Alkandiyl, Oxaalkandiyl, Alkendiyl oder Alkindiyl steht,

$R^1$ für jeweils gegebenenfalls substituiertes Cycloalkyl, Aryl oder Heterocyclyl steht,

$R^2$ für Wasserstoff oder Alkyl steht und

$R^3$ für Wasserstoff, Halogen oder gegebenenfalls substituiertes Alkyl steht,

gefunden.

Die Verbindungen der Formel (I) können gegebenenfalls in Abhängigkeit von der Art der Gruppierung A als geometrische oder als optische Isomere oder als Isomerengemische unterschiedlicher Zusammensetzung vorliegen. Sowohl die reinen Isomeren als auch die Isomerengemische werden als erfindungsgemäß beansprucht.

Weiterhin wurde gefunden, daß man die neuen Cyanothiazolderivate der Formel (I) erhält, wenn man halogenierte Cyanothiazole der allgemeinen Formel (II)

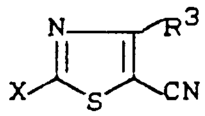

(II)

in welcher

$R^3$ die oben angegebene Bedeutung hat und

X für Halogen steht,

mit Aminen der allgemeinen Formel (III)

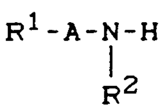

(III)

in welcher

A, $R^1$ und $R^2$ die oben angegebene Bedeutung haben,

gegebenenfalls in Gegenwart eines Säureakzeptors und gegebenenfalls in Gegenwart eines Verdünnungsmittels umsetzt.

Ferner wurde gefunden, daß die neuen Cyanothiazolderivate der Formel (I) interessante herbizide Wirksamkeit aufweisen.

Überraschenderweise zeigen die erfindungsgemäßen Verbindungen der Formel (I) erheblich stärkere Wirkung gegen Unkräuter, als die bekannte Verbindung (S)-4-Amino-5-cyano-2-(1-phenyl-ethylamino)-thiazol, einer nach Struktur und Wirkungsprofil ähnlichen Substanz.

Die in der Definition der Gruppierungen in Formel (I) genannten aliphatischen Ketten, wie Alkyl, Alkandiyl, Oxaalkandiyl, Alkendiyl und Alkindiyl sind jeweils geradkettig oder verzweigt. Diese Ketten enthalten vorzugsweise bis zu 10, insbesondere bis zu 6 Kohlenstoffatome. Als Beispiele für diese Gruppierungen seien genannt: Methyl, Ethyl, Propyl, Isopropyl, Butyl, Isobutyl, sec-Butyl, tert-Butyl, Pentyl, Isopentyl, sec-Pentyl, tert-Pentyl, Hexyl, Isohexyl, sec-Hexyl und tert-Hexyl;

Methandiyl ($-CH_2-$), Ethan-1,1-diyl

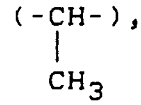

Ethan-1,2-diyl ($-CH_2-CH_2-$), Propan-1,1-diyl

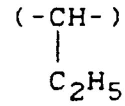

Propan-1,2-diyl

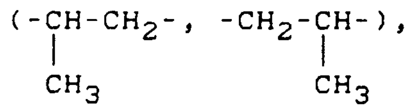

Propan-1,3-diyl ($-CH_2-CH_2-CH_2-$), Butan-1,1-diyl

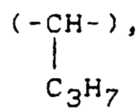

1-Methyl-propan-1,3-diyl

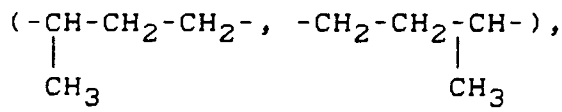

2-Methyl-propan-1,3-diyl

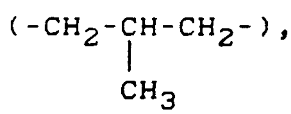

1,2-Dimethyl-ethan-1,2-diyl

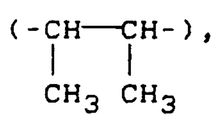

1,1-Dimethyl-propan-1,3-diyl

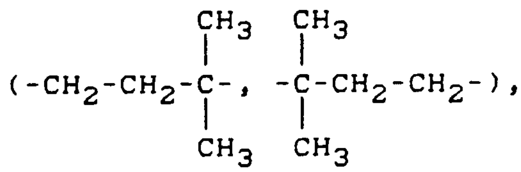

Oxaethandiyl ($-O-CH_2-$), 1-Oxa-propan-1,3-diyl ($-O-CH_2-CH_2-$), 3-Methyl-1-oxa-propan-1,3-diyl

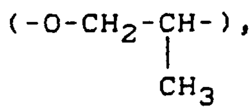

3,3-Dimethyl-1-oxa-propan-1,3-diyl

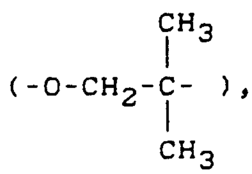

4-Methyl-1-oxa-butan-1,4-diyl

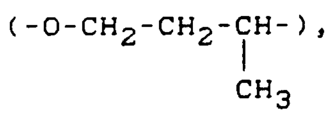

4,4-Dimethyl-1-oxa-butan-1,4-diyl

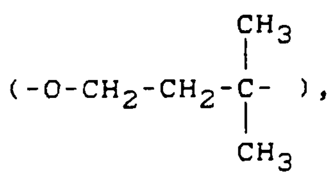

Ethen-1,2-diyl ($-CH=CH-$), Propen-1,3-diyl ($-CH_2-CH=CH-$, $-CH=CH-CH_2-$), 3-Methyl-propen-1,3-diyl

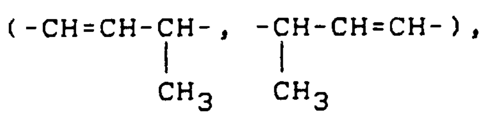

3,3-Dimethyl-propen-1,3-diyl

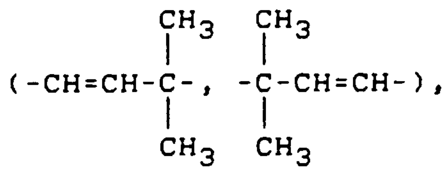

Propin-1,3-diyl ($-CH_2-C\equiv C-$, $-C\equiv C-CH_2-$), 3-Methyl-propin-1,3-diyl

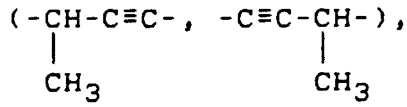

3,3-Dimethyl-propin-1,3-diyl

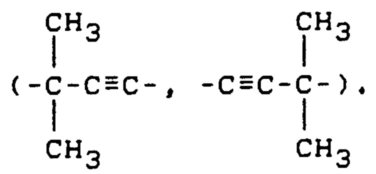

Gegebenenfalls substituiertes Alkyl ($R^3$) in Formel (I) kann als Substituenten vorzugsweise Halogene, insbesondere Fluor und/oder Chlor, oder geradkettige oder verzweigte Alkoxygruppen mit bis zu 4 Kohlenstoffatomen, insbesondere Methoxy oder Ethoxy, welche ihrerseits durch Fluor und/oder Chlor substituiert sein können, enthalten.

Gegebenenfalls substituiertes Cycloalkyl ($R^1$) enthält vorzugsweise 3 bis 8, insbesondere 5 oder 6 Ring-Kohlenstoffatome und kann als Substituenten vorzugsweise Halogene, insbesondere Fluor, Chlor oder Brom, und/oder geradkettiges oder verzweigtes Alkyl mit bis zu 4 Kohlenstoffatomen, insbesondere Methyl, enthalten.

Aryl ($R^1$) enthält vorzugsweise 6 oder 10 Kohlenstoffatome und kann vorzugsweise Substituenten aus der Reihe Halogen, Cyano, Nitro, Amino, Hydroxy, Carboxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils bis zu 6 Kohlenstoffatomen und gegebenenfalls bis zu 9 Halogenatomen, Phenyl, Phenoxy, Cyclohexyl oder gegebenenfalls durch Fluor und/oder Chlor bis zu vierfach substituiertes Alkylendioxy mit 1 oder 2 Kohlenstoffatomen enthalten.

Heterocyclyl ($R^1$) enthält vorzugsweise 2 bis 6 Kohlenstoffatome, gegebenenfalls 1 Sauerstoff- oder Schwefelatom und gegebenenfalls 1 bis 3 Stickstoffatome und kann vorzugsweise Substituenten aus der Reihe Halogen, Cyano, Nitro, Amino, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils bis zu 6 Kohlenstoffatomen und gegebenenfalls bis zu 9 Halogenatomen, Phenyl, Phenoxy oder Cyclohexyl enthalten.

Die Erfindung betrifft vorzugsweise Verbindungen der Formel (I), in welcher

A für jeweils geradkettiges oder verzweigtes Alkandiyl, Oxaalkandiyl, Alkendiyl oder Alkindiyl mit jeweils bis zu 10 Kohlenstoffatomen steht,

$R^1$ für Cycloalkyl mit 3 bis 8 Kohlenstoffatomen, welches gegebenenfalls durch Halogen und/oder durch Alkyl mit 1 bis 4 Kohlenstoffatomen substituiert ist, für Aryl mit 6 oder 10 Kohlenstoffatomen, welches gegebenenfalls durch Halogen, Cyano, Nitro, Amino, Hydroxy, Carboxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils bis zu 6 Kohlenstoffatomen und gegebenenfalls bis zu 9 Halogenatomen, Phenyl, Phenoxy, Cyclohexyl oder Alkylendioxy mit 1 oder 2 Kohlenstoffatomen (welches gegebenenfalls durch Fluor und/oder Chlor bis zu vierfach substituiert ist) substituiert ist, oder für Heterocyclyl mit 2 bis 6 Kohlenstoffatomen, gegebenenfalls einem Sauerstoff- oder Schwefelatom und gegebenenfalls 1 bis 3 Stickstoffatomen, welche gegebenenfalls durch Halogen, Cyano, Nitro, Amino, Hydroxy, jeweils geradkettiges oder verzweigtes Alkyl, Alkoxy, Alkylthio, Halogenalkyl,

Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils bis zu 6 Kohlenstoffatomen und gegebenenfalls bis zu 9 Halogenatomen, Phenyl, Phenoxy oder Cyclohexyl substituiert ist, steht,

$R^2$ für Wasserstoff oder für geradkettiges oder verzweigtes Alkyl mit 1 bis 6 Kohlenstoffatomen steht und

$R^3$ für Wasserstoff, Halogen oder geradkettiges oder verzweigtes Alkyl mit 1 bis 6 Kohlenstoffatomen steht, welches gegebenenfalls durch Halogen oder durch geradkettiges oder verzweigtes Alkoxy mit bis zu 4 Kohlenstoffatomen substituiert ist, welches durch Fluor und/oder Chlor substituiert sein kann.

Die Erfindung betrifft insbesondere Verbindungen der Formel (I), in welcher

A für jeweils geradkettiges oder verzweigtes Alkandiyl, Oxaalkandiyl, Alkendiyl oder Alkindiyl mit jeweils bis zu 6 Kohlenstoffatomen steht,

$R^1$ für Cycloalkyl mit 5 oder 6 Kohlenstoffatomen, welches gegebenenfalls durch Fluor, Chlor, Brom und/oder durch Alkyl mit 1 bis 4 Kohlenstoffatomen substituiert ist, für Aryl mit 6 oder 10 Kohlenstoffatomen, welches gegebenenfalls durch Fluor, Chlor, Brom, Jod, Cyano, Nitro, Amino, Hydroxy, Carboxy, Methyl, Ethyl, Propyl, Isopropyl, Butyl, Isobutyl, sec-Butyl, tert-Butyl, Methoxy, Ethoxy, Propoxy, Isopropoxy, Methylthio, Ethylthio, Trifluormethyl, Difluormethoxy, Trifluormethoxy, Difluormethylthio, Trifluormethylthio, Methylsulfinyl, Ethylsulfinyl, Methylsulfonyl, Ethylsulfonyl, Trifluormethylsulfinyl, Trifluormethylsulfonyl, Acetyl, Propionyl, Methoxycarbonyl oder Ethoxycarbonyl substiuiert ist, oder für eine Heterocyclylgruppierung aus der Reihe Furyl, Tetrahydrofuryl, Thienyl, Pyridyl und Pyrimidyl, welche gegebenenfalls durch Fluor, Chlor, Brom, Cyano, Nitro, Amino, Hydroxy, Methyl, Ethyl, Propyl, Isopropyl, Butyl, Isobutyl, sec-Butyl, tert-Butyl, Methoxy, Ethoxy, Propoxy, Isopropoxy, Methylthio, Ethylthio, Trifluormethyl, Difluormethoxy, Trifluormethoxy, Difluormethylthio, Trifluormethylthio, Methylsulfinyl, Ethylsulfinyl, Methylsulfonyl, Ethylsulfonyl, Trifluormethylsulfinyl, Trifluormethylsulfonyl, Methoxycarbonyl oder Ethoxycarbonyl substituiert ist, steht,

$R^2$ für Wasserstoff, Methyl, Ethyl, Propyl, Isopropyl, Butyl oder Isobutyl steht und

$R^3$ für Wasserstoff, Chlor, Methyl, Ethyl, Propyl, Isopropyl, Butyl, Isobutyl, Trifluormethyl, Methoxymethyl oder Ethoxymethyl steht.

Beispiele für die erfindungsgemäßen Verbindungen der Formel (I) sind in der nachstehenden Tabelle 1 aufgeführt (vgl. auch die Herstellungsbeispiele).

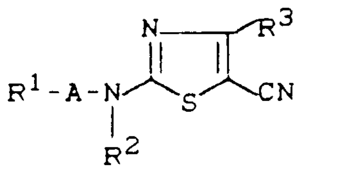

(I)

Tabelle 1: Beispiele für die Verbindungen der Formel (I)

| A | $R^1$ | $R^2$ | $R^3$ |
|---|---|---|---|
| $-CH_2-$ | 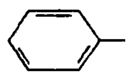 | H | $CH_3$ |
| $-CH_2-$ | 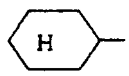 | H | $CH_3$ |
| $-CH_2-$ | 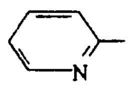 | H | $CH_3$ |
| $-CH_2-$ | 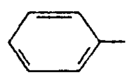 | H | $C_2H_5$ |
| $-CH_2-$ | 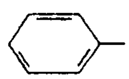 | $CH_3$ | $CH_3$ |
| $-CH(CH_3)-$ | 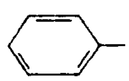 | H | $CH_3$ |
| $-CH_2-CH_2-$ | 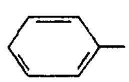 | H | $CH_3$ |
| $-CH(CH_3)-$ | 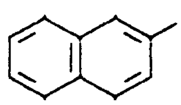 | H | $CH_3$ |
| $-CH(CH_3)-$ | 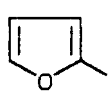 | H | $CH_3$ |
| $-CH(CH_3)-$ | 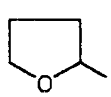 | H | $C_2H_5$ |

Tabelle 1 - Fortsetzung

| A | $R^1$ | $R^2$ | $R^3$ |
|---|---|---|---|
| $-CH(CH_3)-$ | 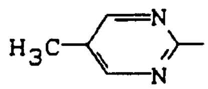 | H | $CH_3$ |
| $-CH(CH_3)-$ | 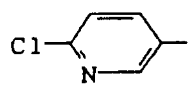 | H | $CH(CH_3)_2$ |
| $-CH_2-CH_2-$ | 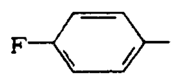 | H | $C_4H_9$ |
| $-CH(CH_3)-$ | 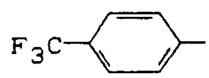 | H | $CH_3$ |
| $-O-CH_2-$ | 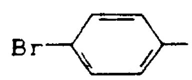 | H | $CH_3$ |
| $-CH_2-CH(CH_3)-$ | 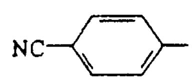 | H | $CH_3$ |
| $-CH(C_2H_5)-$ | 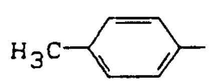 | H | $C_3H_7$ |
| $-CH_2-CH_2-CH(CH_3)-$ | 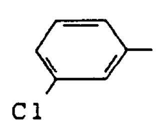 | H | $C_2H_5$ |

Tabelle 1 - Fortsetzung

| A | $R^1$ | $R^2$ | $R^3$ |
|---|---|---|---|
| 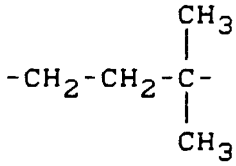 | 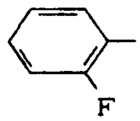 | H | $CH_3$ |
| 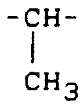 | 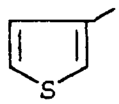 | H | $CH_3$ |
| 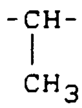 | 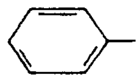 | H | $CF_3$ |
| 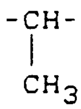 | 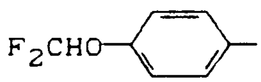 | H | $CH_2OCH_3$ |
| 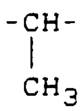 | 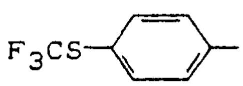 | H | Cl |

Verwendet man für das erfindungsgemäße Verfahren zur Herstellung der neuen Cyanothiazolderivate der Formel (I) beispielsweise 5-Cyano-2,4-dichlor-thiazol und 1-Phenyl-ethylamin als Ausgangsstoffe, so kann der Verlauf des erfindungsgemäßen Verfahrens durch das folgende Formelschema wiedergegeben werden:

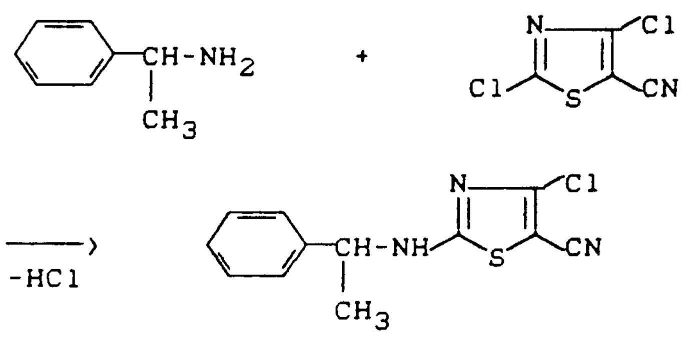


Die beim erfindungsgemäßen Verfahren zur Herstellung von Verbindungen der Formel (I) als Ausgangsstoffe zu verwendenden halogenierten Cyanothiazole sind durch die Formel (II) allgemein definiert.

In Formel (II) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde;

X steht vorzugsweise für Chlor.

Die Ausgangsstoffe der Formel (II) sind bekannt und/oder können nach an sich bekannten Verfahren hergestellt werden (vgl. DE-OS 3038608).

Die beim erfindungsgemäßen Verfahren zur Herstellung von Verbindungen der Formel (I) weiter als Ausgangsstoffe zu verwendenden Amine sind durch die Formel (III) allgemein definiert.

In Formel (III) haben A, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^1$ und $R^2$ angegeben wurden.

Die Ausgangsstoffe der Formel (III) sind bekannt und/oder können nach an sich bekannten Verfahren hergestellt werden (vgl. J. Med. Chem. 25 (1982), 1363-1370; Tetrahedron Lett. 29 (1988), 223-224; Chem. Ber. 117 (1984), 856-858; DE-OS 3426919; EP-A 237305; J. Am. Chem. Soc. 71 (1949), 3482-3485; Tetrahedron Lett. 27 (1986), 3957-3960; Bull. Soc. Chim. France 1974 (3-4, Pt. 2), 615-622; Tetrahedron Lett. 31 (1990), 2661-2664; J. Med. Chem. 13 (1970), 1249-1250).

Das erfindungsgemäße Verfahren zur Herstellung der neuen Cyanothiazolderivate der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden.

Im allgemeinen arbeitet man bei Temperaturen zwischen 0 °C und 150 °C, vorzugsweise bei Temperaturen zwischen 20 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern, insbesondere in monokotylen Kulturen, vor allem im Nachauflauf-Verfahren.

In gewissem Umfang zeigen sie auch fungizide Wirkung, beispielsweise gegen Pyricularia oryzae an Reis, teilweise auch insektizide Wirkung, vor allem gegen Blattinsekten.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennahrstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-

(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxyl-phenoxy}-propansäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin(TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

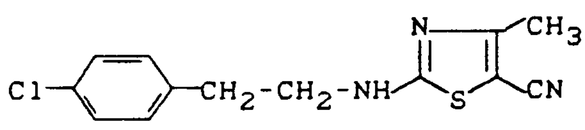

Eine Mischung aus 1,5 g (0,01 Mol) 2-(4-Chlor-phenyl)-ethylamin, 1,6 g (0,01 Mol) 2-Chlor-5-cyano-4-methylthiazol, 5 g Kaliumcarbonat und 50 ml Acetonitril wird 18 Stunden unter Rückfluß erhitzt und anschließend eingeengt. Der Rückstand wird mit Wasser/Chloroform geschüttelt, die organische Phase abgetrennt, mit Natriumsulfat getrocknet und über Kieselgel filtriert. Das Filtrat wird über eine Kieselgelsäule zunächst mit Dichlormethan, dann mit Essigsäureethylester eluiert. Das erste Eluat enthält N,N-Bis-(5-cyano-4-methyl-thiazol-2-yl)-2-(4-chlor-phenyl)-ethylamin, das zweite Eluat das gewünschte Produkt der obigen Formel.

Man erhält 1,1 g (40% der Theorie) N-(5-Cyano-4-methylthiazol-2-yl)-2-(4-chlor-phenyl)-ethylamin vom Schmelzpunkt 178 °C.

Analog Beispiel 1 und gemäß der allgemeinen Beschreibung des erfindungsgemäßen Herstellungsverfahrens können beispielsweise auch die in der nachstehenden Tabelle 2 aufgeführten Verbindungen der Formel (I) hergestellt werden.

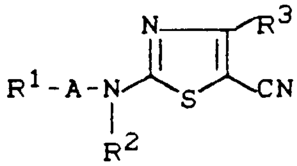

(I)

Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

Tabelle 2: Herstellungsbeispiele für die Verbindungen der Formel (I)

| Bsp.-Nr. | A | $R^1$ | $R^2$ | $R^3$ | Schmelzpunkt (°C) oder $^1H$-NMR |
|---|---|---|---|---|---|
| 2 | (S)-CH-<br>$CH_3$ | 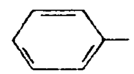 | H | $CH_3$ | δ = 1,62 (d) |
| 3 | (R)-CH-<br>$CH_3$ | 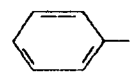 | H | $CH_3$ | δ = 1,62 (d) |
| 4 | (R/S)-CH-<br>$CH_3$ | 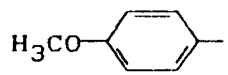 | H | $CH_3$ | δ = 3,82 (s) |
| 5 | (R/S)-CH-<br>$CH_3$ | 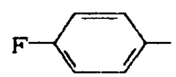 | H | $CH_3$ | δ = 1,59 (d) |
| 6 | (R/S)-CH-<br>$CH_3$ | 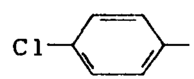 | H | $CH_3$ | δ = 1,58 (d) |
| 7 | (R/S)-CH-<br>$CH_3$ | 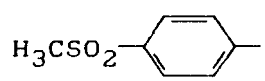 | H | $CH_3$ | δ = 3,22 (s) |

<u>Tabelle 2</u> - Fortsetzung

| Bsp.-Nr. | A | $R^1$ | $R^2$ | $R^3$ | Schmelzpunkt (°C) oder $^1$H-NMR |
|---|---|---|---|---|---|
| 8 | (S)-CH-<br>$CH_3$ | 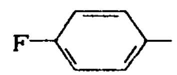 | H | $CH_3$ | δ = 1,59 (d) |
| 9 | (R/S)-CH-<br>$CH_3$ | 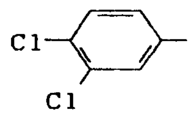 | H | $CH_3$ | δ = 1,58 (d) |
| 10 | (S)-CH-<br>$CH_3$ | 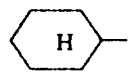 | H | $CH_3$ | Fp = 96° C |
| 11 | (R/S)-CH-<br>$CH_3$ | 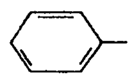 | $CH_3$ | $CH_3$ | δ = 1,62 (d) |
| 12 | (R/S)-CH-<br>$C_2H_5$ | 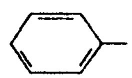 | H | $CH_3$ | δ = 0,95 (t) |
| 13 | (R/S)-CH-<br>$CH_3$ | 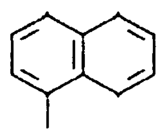 | H | $CH_3$ | Fp = 85° C |

<u>Tabelle 2</u> - Fortsetzung

| Bsp.-Nr. | A | $R^1$ | $R^2$ | $R^3$ | Schmelzpunkt (°C) oder $^1$H-NMR |
|---|---|---|---|---|---|
| 14 | (S)-CH-<br>\|<br>$C_2H_5$ | 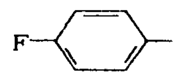 | H | $CH_3$ | δ = 0,95 (t) |
| 15 | (S)-CH-<br>\|<br>$CH_3$ | 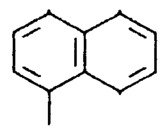 | H | $CH_3$ | δ = 1,78 (d) |
| 16 | (R/S)-$CH_2$-$CH_2$-CH-<br>\|<br>$CH_3$ | 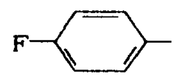 | H | $CH_3$ | δ = 3,5 (q) |
| 17 | $CH_3$<br>\|<br>-$CH_2$-$CH_2$-C-<br>\|<br>$CH_3$ | 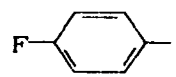 | H | $CH_3$ | δ = 1,45 (s) |
| 18 | (S)-CH-<br>\|<br>$CH_3$ | 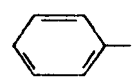 | H | Cl | |

Tabelle 2 - Fortsetzung

| Bsp.-Nr. | A | $R^1$ | $R^2$ | $R^3$ | Schmelzpunkt (°C) oder $^1$H-NMR |
|---|---|---|---|---|---|
| 19 | (R/S)-CH($C_3H_7$)- | 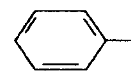 | H | $CH_3$ | Fp = 100° C |
| 20 | $-CH_2-$ | 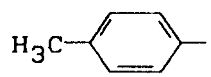 | H | $CH_3$ | Fp = 160° C |
| 21 | $-CH_2-CH_2-$ | 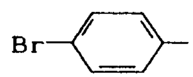 | H | $CH_3$ | Fp = 168° C |
| 22 | $-CH_2-$ | 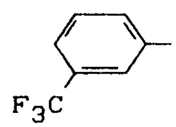 | H | $CH_3$ | Fp = 195° C |
| 23 | $-(CH_2)_3-$ | 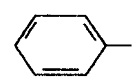 | H | $CH_3$ | Fp = 114° C |
| 24 | $-(CH_2)_4-$ | 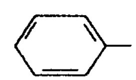 | H | $CH_3$ | Fp = 70° C |

<u>Tabelle 2</u> - Fortsetzung

| Bsp.-Nr. | A | $R^1$ | $R^2$ | $R^3$ | Schmelzpunkt (°C) oder $^1H$-NMR |
|---|---|---|---|---|---|
| 25 | (R/S)-$CH_2$-$CH_2$-CH-<br>\|<br>$CH_3$ | 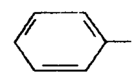 | H | $CH_3$ | (Öl) |
| 26 | (R/S)-CH-$CH_2$-<br>\|<br>$CH_3$ | 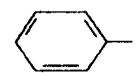 | H | $CH_3$ | Fp = 99° C |
| 27 | (R/S)-CH-<br>\|<br>$CH_3$ | 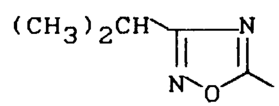 | H | $CH_3$ | Fp = 70° C |
| 28 | (R/S)-CH-<br>\|<br>$CH_3$ | 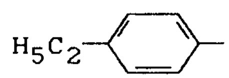 | H | $CH_3$ | Fp = 115° C |
| 29 | (R/S)-CH-<br>\|<br>$CH_3$ | 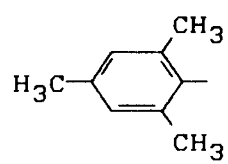 | H | $CH_3$ | Fp = 169° C |

Tabelle 2 - Fortsetzung

| Bsp.-Nr. | A | $R^1$ | $R^2$ | $R^3$ | Schmelzpunkt (°C) oder $^1$H-NMR |
|---|---|---|---|---|---|
| 30 | (R/S)-CH-<br>$CH_3$ | 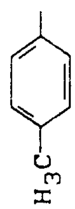 | H | $CH_3$ | (Öl) |
| 31 | (R/S)-CH-<br>$CH_3$ | 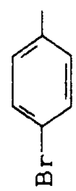 | H | $CH_3$ | (Öl) |
| 32 | (R/S)-CH-<br>$CH_3$ | 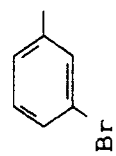 | H | $CH_3$ | (Öl) |

Anwendungsbeispiele:

In den nachstehenden Anwendungsbeispielen wurde die folgende Verbindung als Vergleichssubstanz herangezogen:

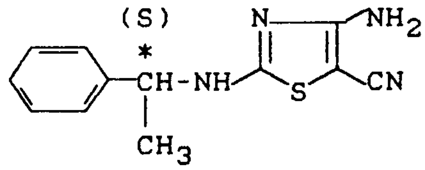

(A)

(S)-4-Amino-5-cyano-2-(1-phenyl-ethylamino)-thiazol (bekannt aus EP-A 328954, Bsp. 1).

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)

100 % = totale Vernichtung

Eine deutliche Überlegenheit in der Wirksamkeit gegenüber dem Stand der Technik zeigt in diesem Test z.B. die Verbindung gemäß Herstellungsbeispiel 2.

**Patentansprüche**

**1.** Cyanothiazolderivate der allgemeinen Formel (I)

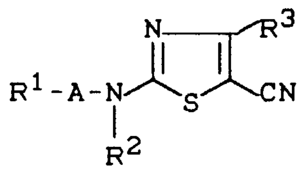

(I)

dadurch gekennzeichnet, daß

A für Alkandiyl, Oxaalkandiyl, Alkendiyl oder Alkindiyl steht,

$R^1$ für jeweils gegebenenfalls substituiertes Cycloalkyl, Aryl oder Heterocyclyl steht,

$R^2$ für Wasserstoff oder Alkyl steht und

$R^3$ für Wasserstoff, Halogen oder gegebenenfalls substituiertes Alkyl steht.

**2.** Cyanothiazolderivate der allgemeinen Formel (I) gemäß Anspruch 1, dadurch gekennzeichnet, daß

A für jeweils geradkettiges oder verzweigtes Alkandiyl, Oxaalkandiyl, Alkendiyl oder Alkindiyl mit jeweils bis zu 10 Kohlenstoffatomen steht,

$R^1$ für Cycloalkyl mit 3 bis 8 Kohlenstoffatomen, welches gegebenenfalls durch Halogen und/oder durch Alkyl mit 1 bis 4 Kohlenstoffatomen substituiert ist, für Aryl mit 6 oder 10 Kohlenstoffatomen, welches gegebenenfalls durch Halogen, Cyano, Nitro, Amino, Hydroxy, Carboxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils bis zu 6 Kohlenstoffatomen und gegebenenfalls bis zu 9 Halogenatomen, Phenyl, Phenoxy, Cyclohexyl oder Alkylendioxy mit 1 oder 2 Kohlenstoffatomen (welches gegebenenfalls durch Fluor und/oder Chlor bis zu vierfach

substituiert ist) substituiert ist, oder für Heterocyclyl mit 2 bis 6 Kohlenstoffatomen, gegebenenfalls einem Sauerstoff- oder Schwefelatom und gegebenenfalls 1 bis 3 Stickstoffatomen, welche gegebenenfalls durch Halogen, Cyano, Nitro, Amino, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils bis zu 6 Kohlenstoffatomen und gegebenenfalls bis zu 9 Halogenatomen, Phenyl, Phenoxy oder Cyclohexyl substituiert ist, steht,

$R^2$ für Wasserstoff oder für geradkettiges oder verzweigtes Alkyl mit 1 bis 6 Kohlenstoffatomen steht und

$R^3$ für Wasserstoff, Halogen oder geradkettiges oder verzweigtes Alkyl mit 1 bis 6 Kohlenstoffatomen steht, welches gegebenenfalls durch Halogen oder durch geradkettiges oder verzweigtes Alkoxy mit bis zu 4 Kohlenstoffatomen substituiert ist, welches durch Fluor und/oder Chlor substituiert sein kann.

**3.** Cyanothiazolderivate der allgemeinen Formel (I) gemäß Anspruch 1, dadurch gekennzeichnet, daß

A für jeweils geradkettiges oder verzweigtes Alkandiyl, Oxaalkandiyl, Alkendiyl oder Alkindiyl mit jeweils bis zu 6 Kohlenstoffatomen steht,

$R^1$ für Cycloalkyl mit 5 oder 6 Kohlenstoffatomen, welches gegebenenfalls durch Fluor, Chlor, Brom und/ oder durch Alkyl mit 1 bis 4 Kohlenstoffatomen substituiert ist, für Aryl mit 6 oder 10 Kohlenstoffatomen, welches gegebenenfalls durch Fluor, Chlor, Brom, Jod, Cyano, Nitro, Amino, Hydroxy, Carboxy, Methyl, Ethyl, Propyl, Isopropyl, Butyl, Isobutyl, sec-Butyl, tert-Butyl, Methoxy, Ethoxy, Propoxy, Isopropoxy, Methylthio, Ethylthio, Trifluormethyl, Difluormethoxy, Trifluormethoxy, Difluormethylthio, Trifluormethylthio, Methylsulfinyl, Ethylsulfinyl, Methylsulfonyl, Ethylsulfonyl, Trifluormethylsulfinyl, Trifluormethylsulfonyl, Acetyl, Propionyl, Methoxycarbonyl oder Ethoxycarbonyl substiuiert ist, oder für eine Heterocyclylgruppierung aus der Reihe Furyl, Tetrahydrofuryl, Thienyl, Pyridyl und Pyrimidyl, welche gegebenenfalls durch Fluor, Chlor, Brom, Cyano, Nitro, Amino, Hydroxy, Methyl, Ethyl, Propyl, Isopropyl, Butyl, Isobutyl, sec-Butyl, tert-Butyl, Methoxy, Ethoxy, Propoxy, Isopropoxy, Methylthio, Ethylthio, Trifluormethyl, Difluormethoxy, Trifluormethoxy, Difluormethylthio, Trifluormethylthio, Methylsulfinyl, Ethylsulfinyl, Methylsulfonyl, Ethylsulfonyl, Trifluormethylsulfinyl, Trifluormethylsulfonyl, Methoxycarbonyl oder Ethoxycarbonyl substituiert ist, steht,

$R^2$ für Wasserstoff, Methyl, Ethyl, Propyl, Isopropyl, Butyl oder Isobutyl steht und

$R^3$ für Wasserstoff, Chlor, Methyl, Ethyl, Propyl, Isopropyl, Butyl, Isobutyl, Trifluormethyl, Methoxymethyl oder Ethoxymethyl steht.

**4.** Cyanothiazolderivate der allgemeinen Formel (I) gemäß Anspruch 1, dadurch gekennzeichnet, daß Methandiyl ($-CH_2-$), Ethan-1,1-diyl

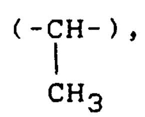

Ethan-1,2-diyl ($-CH_2-CH_2-$), Propan-1,1-diyl

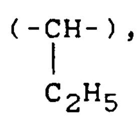

Propan-1,2-diyl

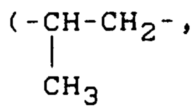

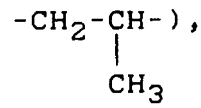

Propan-1,3-diyl ($-CH_2-CH_2-CH_2-$), Butan-1,1-diyl

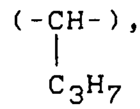

1-Methyl-propan-1,3-diyl

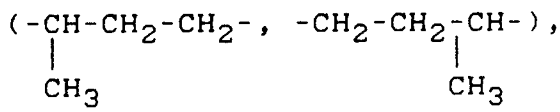

2-Methyl-propan-1,3-diyl

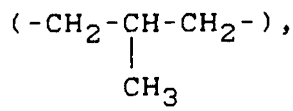

1,2-Dimethyl-ethan-1,2-diyl

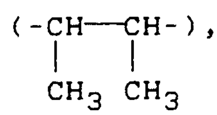

1,1-Dimethyl-propan-1,3-diyl

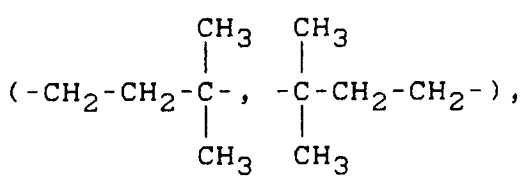

Oxaethandiyl ($-O-CH_2-$),
1-Oxa-propan-1,3-diyl ($-O-CH_2-CH_2-$),
3-Methyl-1-oxa-propan-1,3-diyl

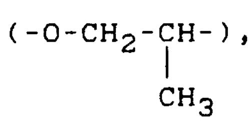

3,3-Dimethyl-1-oxa-propan-1,3-diyl

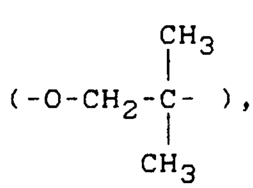

4-Methyl-1-oxa-butan-1,4-diyl

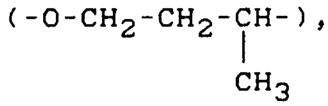

4,4-Dimethyl-1-oxa-butan-1,4-diyl

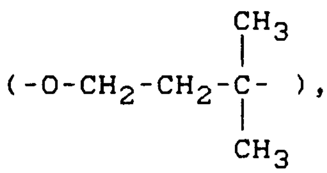

Ethen-1,2-diyl (-CH = CH-),
Propen-1,3-diyl ($-CH_2$-CH = CH-, -CH = CH-$CH_2$-),
3-Methyl-propen-1,3-diyl

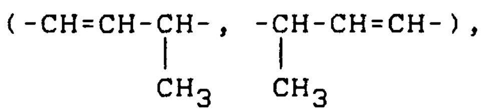

3,3-Dimethyl-propen-1,3-diyl

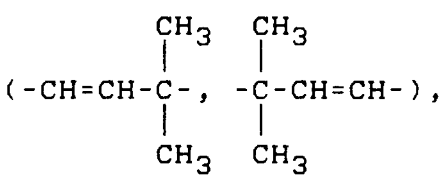

Propin-1,3-diyl ($-CH_2-C\equiv C-$, $-C\equiv C-CH_2-$),
3-Methyl-propin-1,3-diyl

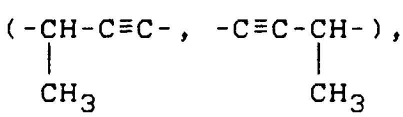

3,3-Dimethyl-propin-1,3-diyl

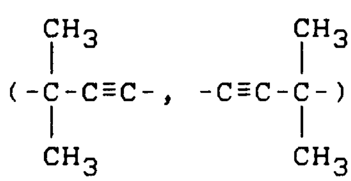 steht,

$R^1$ für Cycloalkyl mit 5 oder 6 Kohlenstoffatomen, welches gegebenenfalls durch Fluor, Chlor, Brom und/ oder durch Alkyl mit 1 bis 4 Kohlenstoffatomen substituiert ist, für Aryl mit 6 oder 10 Kohlenstoffatomen, welches gegebenenfalls durch Fluor, Chlor, Brom, Jod, Cyano, Nitro, Amino, Hydroxy, Carboxy, Methyl, Ethyl, Propyl, Isopropyl, Butyl, Isobutyl, sec-Butyl, tert-Butyl, Methoxy, Ethoxy, Propoxy, Isopropoxy, Methylthio, Ethylthio, Trifluormethyl, Difluormethoxy, Trifluormethoxy, Difluormethylthio, Trifluormethylthio, Methylsulfinyl, Ethylsulfinyl, Methylsulfonyl, Ethylsulfonyl, Trifluormethylsulfinyl, Trifluormethylsulfonyl, Acetyl, Propionyl, Methoxycarbonyl oder Ethoxycarbonyl substituiert ist, oder für eine Heterocyclylgruppierung aus der Reihe Furyl, Tetrahydrofuryl, Thienyl, Pyridyl und Pyrimidyl, welche gegebenenfalls durch Fluor, Chlor, Brom, Cyano, Nitro, Amino, Hydroxy, Methyl, Ethyl, Propyl, Isopropyl, Butyl, Isobutyl, sec-Butyl, tert-Butyl, Methoxy, Ethoxy, Propoxy, Isopropoxy, Methylthio, Ethylthio, Trifluormethyl, Difluormethoxy, Trifluormethoxy, Difluormethylthio, Trifluormethylthio, Methylsulfinyl, Ethylsulfinyl, Methylsulfonyl, Ethylsulfonyl, Trifluormethylsulfinyl, Trifluormethylsulfonyl, Methoxycarbonyl oder Ethoxycarbonyl substituiert ist, steht,

**5.** Verfahren zur Herstellung von Cyanothiazolderivaten der Formel (I)

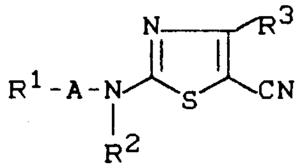

(I)

in welcher
A für Alkandiyl, Oxaalkandiyl, Alkendiyl oder Alkindiyl steht,
$R^1$ für jeweils gegebenenfalls substituiertes Cycloalkyl, Aryl oder Heterocyclyl steht,
$R^2$ für Wasserstoff oder Alkyl steht und
$R^3$ für Wasserstoff, Halogen oder gegebenenfalls substituiertes Alkyl steht,
dadurch gekennzeichnet, daß man halogenierte Cyanothiazole der Formel (II)

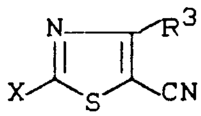

(II)

in welcher
$R^3$ die oben angegebene Bedeutung hat und
X für Halogen steht,
mit Aminen der allgemeinen Formel (III)

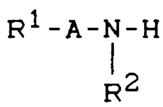

(III)

in welcher
A, $R^1$ und $R^2$ die oben angegebene Bedeutung haben,
gegebenenfalls in Gegenwart eines Säureakzeptors und gegebenenfalls in Gegenwart eines Verdünnungsmittels umsetzt.

**6.** Herbizide Mittel, gekennzeichnet durch einen Gehalt an mindestens einem Cyanothiazolderivat der Formel (I) gemäß Anspruch 1 oder 5.

**7.** Verfahren zur Bekämpfung von unerwünschten Pflanzen, dadurch gekennzeichnet, daß man Cyanothiazolderivate der Formel (I) gemäß Anspruch 1 oder 5 auf die unerwünschten Pflanzen und/oder ihren Lebensraum einwirken läßt.

**8.** Verwendung von Cyanothiazolderivaten der Formel (I) gemäß Anspruch 1 oder 5 zur Bekämpfung von unerwünschten Pflanzen.

**9.** Verfahren zur Herstellung von herbiziden Mitteln, dadurch gekennzeichnet, daß man Cyanothiazolderivate gemäß Anspruch 1 oder 5 mit Streckmitteln und/oder oberflächenaktiven Substanzen vermischt.

Europäisches Patentamt

# EUROPÄISCHER RECHERCHENBERICHT

Nummer der Anmeldung

EP 92 10 5746

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | EP-A-0 328 954 (BAYER AG)<br>* Ansprüche *<br>--- | 1,6-9 | C07D277/56<br>C07D417/06<br>A01N43/78 |
| A | US-A-4 324 899 (MARK D. FRISHBERG)<br>* das ganze Dokument *<br>--- | 1 | |
| A | DE-A-3 038 608 (BAYER AG)<br>* Ansprüche *<br>--- | 1,6-9 | |
| A | JOURNAL OF THE CHEMICAL SOCIETY, PERKIN TRANSACTIONS 1.<br>Nr. 2, 1984, LETCHWORTH GB<br>Seiten 147 - 153;<br>TIMOTHY N. BIRKINSHAW: '2-(N,N-disubstituted amino)thiazoles with electron-withdrawing groups at position 5:preparation and investigation of structural features'<br>* das ganze Dokument *<br>----- | 1 | |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 24 JUNI 1992 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument